# EUROPÄISCHE PATENTSCHRIFT

(11) **EP 3 723 849 B1**
(45) Veröffentlichungstag und Bekanntmachung des Hinweises auf die Patenterteilung: **10.01.2024**
(21) Anmeldenummer: 18810951.6
(22) Anmeldetag: 20.11.2018
(51) Int. Cl.: A61N 1/375, B29C 45/14, B29L 31/00

(54) **VERFAHREN ZUM HERSTELLEN EINES KOPFTEILS EINER IMPLANTIERBAREN MEDIZINISCHEN VORRICHTUNG**
METHOD FOR PRODUCING A HEAD PART OF AN IMPLANTABLE MEDICAL DEVICE
PROCÉDÉ DE FABRICATION D'UNE PARTIE TÊTE D'UN DISPOSITIF MÉDICAL IMPLANTABLE

(30) Priorität: 11.12.2017 DE 102017222364
(43) Veröffentlichungstag der Anmeldung: 21.10.2020
(73) Patentinhaber: Neuroloop GmbH, 79108 Freiburg (DE)
(72) Erfinder: KIMMIG, Fabian, 79110 Freiburg (DE); BORETIUS, Tim, 79110 Freiburg (DE)
(74) Vertreter: Rösler, Uwe
(86) Internationale Anmeldenummer: PCT/EP2018/081922
(87) Internationale Veröffentlichungsnummer: WO 2019/115176

(56) Entgegenhaltungen:
- DE-U1-202013 012 073
- US-A1- 2015 142 092
- US-A1- 2016 100 887
- US-B1- 8 267 708

## Beschreibung

### Technisches Gebiet

Die Erfindung bezieht sich auf ein Verfahren zum Herstellen eines Kopfteils einer implantierbaren medizinischen Vorrichtung, mit einem Kopfteilgehäuse, das eine sacklochartige Ausnehmung besitzt, längs der in koaxialer Anordnung und axial serieller Abfolge wenigstens ein elektrisch leitendes Kontaktringelement sowie ein elektrisch isolierender, elastisch verformbarer Dichtungsring unter axialer Fügekraft gegeneinander kraftschlüssig gefügt sind.

### Stand der Technik

Implantierbare medizinische Vorrichtungen zum Zwecke der elektrischen Stimulation lokaler intrakorporaler Bereiche, kurz implantierbare Impulsgeber, (IPG) beispielsweise für herztherapeutische Defribillations-, Schrittmacher- sowie Resynchronisationsanwendungen, für neurostimulationstherapeutische Maßnahmen, wie bspw. Rückenmarstimulation, Hirnstimulation oder Vagusnervstimulation, um nur einige zu nennen, weisen in aller Regel eine in sich abgeschlossenes Gehäuse auf, in dem Komponenten zur elektrischen Pulsgeneration enthalten sind, so zumindest eine elektrische Energiequelle und eine mit dieser verbundenen elektrischen Schaltungsstruktur. Zudem schließt an das Gehäuse ein sogenanntes Kopfteil an, in dem eine mit der Energiequelle bzw. der elektrischen Schaltungsstruktur elektrisch verbundene, elektrische Kontaktanordnung enthalten ist, in die eine fluiddicht mit dem Kopfteil abschließende Steckeranordnung einfügbar ist, die mit elektrischen Zu- und Ableitungen zum Zwecke der intrakorporalen lokalen Applikation der elektrischen Stimulationssignale sowie gegebenenfalls der Zuführung intrakorporal lokal abgegriffener elektrischer Signale zur Gehäuseseitig vorhandenen elektrischen Schaltungsstruktur, kontaktiert ist.

In der Druckschrift EP 2 134 418 B1 ist ein gattungsgemäßes Kopfteil einer implantierbaren medizinischen Vorrichtung beschrieben, das zwei längs einer Fügenaht zusammenfügbare Kopfteilgehäusehälften umfasst, in die jeweils in serieller Abfolge durch Zwischenwände beabstandet, halbzylinderförmige Ausnehmungen eingebracht sind, in die in jeweils seriell abwechselnder Reihenfolge elektrisch leitende Kontaktringelemente sowie elektrisch isolierende Dichtungsringe eingesetzt sind. Das aus den zwei Kopfteilgehäusehälften zusammengefügte Kopfteil umfasst somit eine Anordnung koaxial zueinander ausgerichteter und elektrisch isolierter Kontaktringelemente, zu deren elektrischen Kontaktierung ein seitlicher Zugang im Kopfteil vorgesehen ist, durch den eine elektrische Steckeranordnung in einen von sämtlichen ringförmigen Kontaktringelementen umfassten Hohlraum fluiddicht einfügbar ist.

Die Druckschrift DE 10 2012 010 901 A1 offenbart ein Verfahren zum Positionieren und Halten von elektrischen Kontakten und Dichtungen innerhalb eines Kopfteils zur elektrischen Kontaktierung an eine medizinische implantierbare Vorrichtung. In das aus einem bioverträglichen und elektrisch isolierenden Material bestehende Kopfteilgehäuse ist einseitig eine sacklochartige Bohrung eingebracht, in der in abwechselnder Reihenfolge elektrisch leitende Kontaktringe und ringförmige Dichtelemente eingebracht sind, die gemeinsam einen Hohlraum umfassen, in den eine stiftförmige Steckeranordnung einfügbar ist. Jeder der einzelnen ringförmigen Kontaktringe ist innerhalb des Kopfteils ist über eine elektrische Verbindungsleitung mit innerhalb des Gehäuses der medizinische implantierbare Vorrichtung befindlichen elektrischen Komponenten verbunden.

Zum Zwecke einer möglichst dauerhaften fluiddichten Abdichtung sowie auch elektrischen Isolierung der einzelnen elektrischen Kontaktringelemente untereinander gilt es, die abwechselnde Abfolge von elektrischen Kontaktringelementen und Dichtungsringen mit einer möglichst großen axialen Fügekraft innerhalb des Kopfteils zu fixieren. Hierzu bedarf es aufwändiger Montagemaßnahmen, die zumeist nur manuell und unter Aufbringung beachtlicher Fingerfertigkeit durchgeführt werden können.

In der Druckschrift DE 20 2013 012 073 U1 ist eine Steckerbohrung-Modulbaugruppe offenbart, zu deren Montage eine Anzahl von Kontaktringen und Dichtelementen in abwechselnder Reihenfolge längs eines stiftförmigen Montagewerkzeuges angeordnet sind. Mittels einer Spannvorrichtung werden sämtliche längs des Montagewerkzeuges aufsitzenden Kontaktringe und Dichtelemente unter Aufbringung einer axialen Fügekraft gegeneinander verspannt. Zum Zwecke der Konservierung der Fügekraft dient ein mittels einer Madenschraube axialfest auf dem Montagewerkzeug aufsitzendes Hülsenelement, das zusammen mit einem endseitigen Montagewerkzeugkopf die Anordnung aus Kontaktringen und Dichtelementen axial beidseitig begrenzt. In diesem verspannten Zustand erfolgt ein Vergießen der Anordnung mit einer aushärtbaren Vergußmasse, die im erstarrten Zustand die Fügekraft aufnimmt.

### Darstellung der Erfindung

Der Erfindung liegt die Aufgabe zugrunde ein Verfahren zum Herstellen eines Kopfteils einer implantierbaren medizinischen Vorrichtung, mit einem Kopfteilgehäuse, das eine sacklochartige Ausnehmung besitzt, längs der in koaxialer Anordnung und axial serieller Abfolge wenigstens ein elektrisch leitendes Kontaktringelement sowie ein elektrisch isolierender, elastisch verformbarer Dichtungsring mit axialer Fügekraft gegeneinander kraftschlüssig gefügt sind, derart weiterzubilden, so dass der verfahrenstechnische sowie auch zeit- und kostenrelevante Aufwand für die Herstellung sowohl individuell konfektionierter als auch von in großer Stückzahl gefertigter Kopfteile signifikant reduziert werden soll. Von besonderem Interesse ist es überdies, dass die Fertigungsqualität sowie die Fluiddichtigkeit und die damit verbundene Lebensdauer eines Kopfteils höchsten Ansprüchen genügen muss.

Die Lösung der der Erfindung zugrunde liegenden Aufgabe ist im Anspruch 1 angegeben. Den Erfindungsgedanken in vorteilhafter Weise weiterbildende Merkmale sind Gegenstand der Unteransprüche sowie der Beschreibung, insbesondere unter Bezugnahme auf die illustrierten Ausführungsbeispiele zu entnehmen.

Das lösungsgemäße Verfahren zum Herstellen eines Kopfteils einer implantierbaren medizinischen Vorrichtung gemäß den Merkmalen des Oberbegriffes des Anspruches 1 setzt sich aus den folgenden Verfahrensschritten zusammen.

Wenigstens ein elektrisch leitendes Kontaktringelement sowie wenigstens ein elektrisch isolierender, aus elastisch verformbarem Material bestehender Dichtungsring werden längs eines stabförmigen Montagewerkzeuges, das vorzugsweise als Stift, Stange oder Pin ausgebildet ist, angeordnet. Das Anordnen erfolgt durch einfaches Auffädeln vorzugsweise einer Vielzahl von Kontaktringelementen und Dichtungsringen auf das stabförmige Montagewerkzeug unter jeweils einer seriell abwechselnden Reihenfolge zwischen Kontaktringelementen und Dichtungsringen.

Anschließend werden das wenigstens eine Kontaktringelement sowie der wenigstens eine Dichtungsring vermittels einer längs des stabförmigen Montagewerkzeuges orientierten Fügekraft kraftschlüssig mit- bzw. gegeneinander gefügt. Hierdurch schmiegt sich der wenigstens eine elektrisch isolierende und elastisch verformbare Dichtungsring an eine dem Dichtungsring zugewandte Oberfläche des elektrisch leitenden Kontaktringelementes unter Ausbildung einer fluiddichten und ringförmig kraftbeaufschlagten Fügeverbindung an.

Zum Erzeugen der zwischen dem wenigstens einen Kontaktringelement und dem Dichtungsring längs des stabförmigen Montagewerkzeuges axial wirkenden Fügekraft dienen jeweils beidseitig zu dem wenigstens einen Kontaktringelement und dem Dichtungsring längs des Montagewerkzeuges angebrachte Befestigungsmittel, von denen wenigstens ein Befestigungsmittel axial beweglich und lösbar axial fest am Montagewerkzeug fixierbar ist und in Art einer Mutter mit Innengewinde ausgebildet ist, das in Eingriff mit einem längs des Montagewerkzeuges vorgesehenen Außengewinde derart gebracht wird, so dass das in Art einer Mutter ausgebildete Befestigungsmittel durch relatives Verdrehen längs des Außengewindes des Montagewerkzeuges einseitig kraftschlüssig unter Ausbildung der Fügekraft in Eingriff mit dem wenigstens einen Kontaktringelement oder Dichtungsring gebracht wird. Die Vermittels des Gewindeeingriffes zwischen dem Inngewinde des in Art einer Mutter ausgebildeten Befestigungsmittels und dem am Montagewerkzeug angebrachten Außengewinde bedingte, relative Längsverstellbarkeit des Befestigungsmittels längs des Montagewerkzeuges ermöglicht eine feindosierbare Einstellung der Fügekraft, die allein durch Rotation des Montagewerkzeuges relativ zum Befestigungsmittel erzeugt werden kann. Vorzugsweise verfügt das Montagewerkzeug über einen endseitig ausgebildeten Werkzeugkopf mit Werkzeugflansch, bspw. zur lösbar festen Ineingriffnahme mit einem Drehwerkzeug in Form eines Schlitz- oder Kreuzschraubenschlüssels oder eines Innbusschlüssels o.ä..

Das in Art einer Mutter ausgebildete Befestigungsmittel kann in vielerlei Weise konstruktiv ausgebildet sein. Neben einer klassischen Mutter, bietet es sich an ein Plattenelement mit wenigstens einer, das Innengewinde aufweisenden Gewindebohrung vorzusehen. Durch Vorsehen von zwei oder mehr Gewindebohrungen innerhalb eines Plattenelementes kann die Anzahl von separat nebeneinander angeordneten Anordnungen mit jeweils einer koaxialen Abfolge von Kontaktringelementen und Dichtungsringen innerhalb eines Kopfteils beliebig vorgebbar räumlich zueinander angeordnet und skaliert werden. Auf diese Weise können Kopfteile mit mehreren elektrischen Anschlußbuchsen ausgebildet werden und dies in einer kompakten und leicht zu realisierenden Bauweise. Zudem ermöglicht die Verwendung eines Plattenelementes eine konkret vorgebbare räumliche Relativanordnung zwischen zwei oder mehr innerhalb eines Kopfteils angeordneter elektrischer Anschlußbuchsen.

Durch eine aufeinander abgestimmte Dimensionierung der axialen Gewindelängen von Innen- und Außengewinde und eine damit verbundene maximal vorgebbare Eindrehung des Montagewerkzeuges in das Innengewinde des Befestigungsmittels lässt sich eine definiert vorgebbare maximale Fügekraft zwischen den Kontaktringelementen und Dichtungsringen konstruktiv vorgeben, wodurch der Montageprozess sicher und reproduzierbar durchführbar ist.

In einer bevorzugten Ausführungsform weist das stabförmige Montagewerkzeug kopfseitig einen axialfest mit diesem verbundenen mechanischen Anschlag auf, der lösbar fest oder einstückig mit dem Montagewerkzeug verbunden ist, an den die axiale Stapelanordnung, bestehend aus dem wenigstens einen Kontaktringelement sowie dem Dichtungsring, einseitig abstützend anliegt. Dem mechanischen Anschlag zur axialen Stapelanordnung gegenüberliegend ist das längs des stabförmigen Montagewerkzeuges bewegbare und mit dem Montagewerkzeug axial lösbar fest verbindbare Befestigungsmittel in Form einer Mutter angebracht.

Das vorstehend mit dem wenigstens einen elektrisch leitenden Kontaktelement sowie dem wenigstens einen Dichtungsring vorkonfektionierte Montagewerkzeug stellt ein einheitlich handhabbares Halbzeug dar, das in einem weiteren Verfahrensschritt mit einer Vergussmasse ummantelt wird. Hierzu werden zumindest das längs des stabförmigen Montagewerkzeuges angeordnete Kontaktringelement und der wenigstens eine Dichtungsring, die einander kraftschlüssig gefügt sind, mit einer erstarrungsfähigen, in fließfähiger Form vorliegenden Vergussmasse ummantelt. Aufgrund der gegenseitigen, kraftschlüssigen und fluiddichten Fügung des wenigstens einen Kontaktringelementes mit dem Dichtungsring vermag die fließfähige Vergussmasse nicht in den von dem wenigstens einen Kontaktringelement und Dichtungsring gemeinsam umschlossenen Innenraum einzudringen.

Als Vergussmasse dient vorzugsweise eine biokompatible Kunststoff- oder Harzmasse, vorzugsweise Epoxide, die nach kurzer Zeit vollständig unter Ausbildung eines formstabilen Körpers erstarrt. Nach Erstarren der Vergussmasse wird schließlich das stabförmige Montagewerkzeug von dem wenigstens einen Kontaktringelement sowie dem Dichtungsring gelöst und entfernt. Die zwischen dem wenigstens einen Kontaktringelement und dem Dichtungsring wirkende axiale Fügekraft bleibt unverändert erhalten und wird von der erstarrten Vergussmasse in Form einer zumindest einen Teil des Kopfteilgehäuses bildenden, formstabilen Matrix vollständig abgestützt. Das in Art einer Mutter ausgebildete Befestigungsmittel verbleibt in der erstarrten Vergußmasse.

Um das Lösen und Entfernen des Montagewerkzeuges aus dem Kopfteil zu erleichtern, wird das durch das Innengewinde des in Art einer Mutter ausgebildeten Befestigungsmittels überstehende Ende des Montagewerkzeuges vor dem Vergießen mit der fließfähigen Vergußmasse mit einer Schutzmasse, vorzugsweise mit Silikon o.ä. benetzt, die verhindert, dass die erstarrte Vergußmasse eine feste Anhaftung mit dem überstehenden Montagewerkzeugendabschnitt ausbildet.

### Kurze Beschreibung der Erfindung

Die Erfindung wird nachstehend ohne Beschränkung des allgemeinen Erfindungsgedankens anhand von Ausführungsbeispielen unter Bezugnahme auf die Zeichnungen exemplarisch beschrieben. Es zeigen:
- Fig. 1: schematische Darstellung einer Axialstapelanordnung aus Kontaktringelementen und Dichtungsringen längs eines stabförmig ausgebildeten Montagewerkzeuges,
- Fig. 2a, b: Detailansichten zur Ausbildung eines lösbar axial fest anbringbarem Befestigungsmittels längs des stabförmigen Montagewerkzeuges,
- Fig. 3: Verfahrensschritt der Ummantelung der auf dem stabförmigen Montagewerkzeug aufgebrachten stapelförmigen Anordnung aus Kontaktringelementen und Dichtungsringen mit einer Vergussmasse sowie
- Fig. 4: Darstellung der erstarrten Vergussmasse mit eingeschlossener Stapelanordnung bestehend aus Kontaktringelementen und Dichtungsringen.

### Wege zur Ausführung der Erfindung, gewerbliche Verwendbarkeit

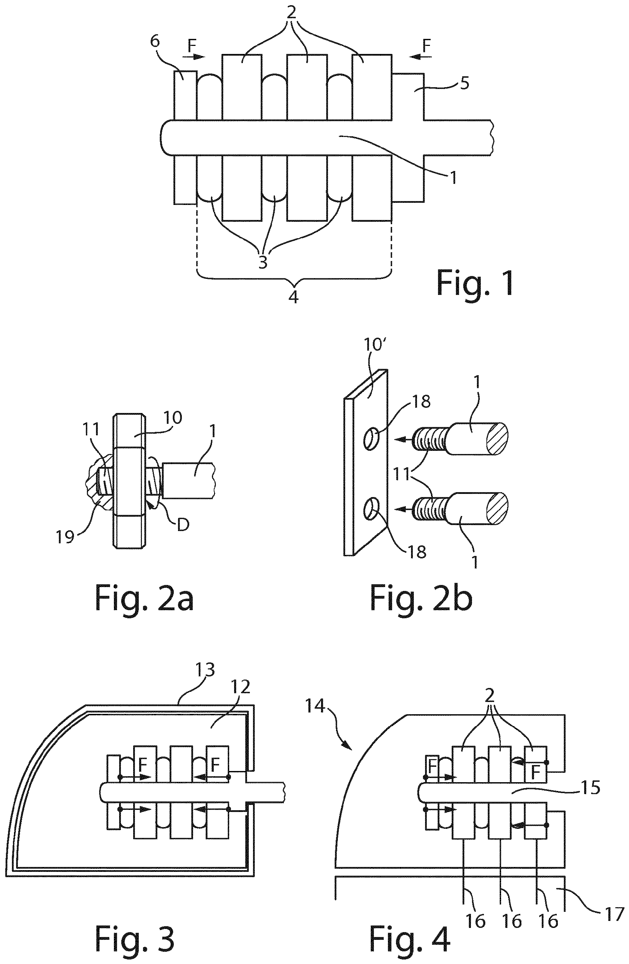

Figur 1 illustriert schematisch ein stabförmig ausgebildetes Montagewerkzeug 1, längs dem in axial serieller abwechselnder Abfolge elektrisch leitende Kontaktringelemente 2 und jeweils dazwischen befindliche elektrisch isolierende, aus elastomeren Material gefertigte Dichtungsringe 3 angeordnet sind. Beidseitig zu der aus der abwechselnden Abfolge aus Kontaktringelementen 2 und Dichtungsringen 3 zusammengesetzten axialen Stapelanordnung 4 sind jeweils längs des stabförmigen Montagewerkzeuges 1 Befestigungsmittel 5, 6 angebracht. Im Falle des in Figur 1 dargestellten Befestigungsmittels 5 handelt es sich um einen mit dem ansonsten stabförmig ausgebildeten Montagewerkzeug 1 teller- bzw. scheibenartig ausgebildeten, einstückig mit dem Montagwerkzeug verbundenen, mechanischen Anschlag, an dem die axiale Stapelanordung 4 einseitig unmittelbar angrenzt. Das zu diesem längs der axialen Stapelanordnung 4 gegenüberliegend angeordnete Befestigungsmittel 6 ist axial beweglich längs des stabförmigen Montagewerkzeuges 1 ausgebildet und verfügt überdies über einen Arretierungsmechanismus, der das Befestigungsmittel 6 axial fest relativ zum stabförmigen Montagewerkzeug 1 zu fixieren vermag.

Die Figuren 2a und b zeigen bevorzugte Ausbildungsformen zur Realisierung des axial beweglichen und axial fest arretierbaren Befestigungsmittels 6. Figur 2a stellt eine Mutter 10 mit Innengewinde dar, die in Eingriff mit einem endseitig längs des stabförmigen Montagewerkzeuges 1 vorgesehenen Außengewindes 11 steht. Das als Mutter 10 ausgebildete Befestigungsmittel 6 muss nicht notwendigerweise als klassische Mutter ausgebildet sein, vielmehr können beliebige Körper oder Körperformen mit einem Innengewinde 18 als Befestigungsmittel 6 dienen. In Figur 2b ist als Befestigungsmittel 6 ein Plattenelement 10' illustriert, das zwei nebeneinander angeordnete Öffnungen jeweils mit Innengewinde 11 vorsieht, in die jeweils ein Montagewerkzeug 1, über dessen Außengewinde 11 in Eingriff bringbar ist. Das Plattenelement 10' kann beliebig zwei- oder dreidimensional geformt und dimensioniert sein. Überdies kann eine beliebige Anzahl von Öffnungen mit Innengewinde 11 in das Plattenelement 10' eingebracht sein. Hierdurch lassen sich mehrere Anordnungen jeweils aus koaxial angeordneten Kontaktringelementen 2 und Dichtungsringen 3 innerhalb eines Kopfteils realisieren, die jeweils als elektrische Einsteckbuchsen für Steckereinheiten dienen. Je nach Ausbildung des Plattenelementes 10' sowie Anzahl und Anordnung von mit Innengewinden 11 versehenen Öffnungen innerhalb des Plattenelementes 10' lassen sich beliebig komplexe Kopfteile ohne großen Herstellungs- und Montageaufwand realisieren.

Zum Aufbringen einer axial zum stabförmigen Montagewerkzeug 1 orientierten Fügekraft, durch die die abwechselnde Abfolge von Kontaktringelementen 2 und Dichtungsringen 3 kraftschlüssig mit- bzw. gegeneinander gefügt werden, gilt es das Montagewerkzeug 1 relativ zur Mutter 10 bzw. zum Plattenelement 10' zu drehen, bspw. durch vollständiges Eindrehen des Montagewerkzeug-seitigen Außengewindes 11 in das Innengewinde 11, wodurch sich eine längs der auf dem Montagewerkzeug aufsitzenden Kontaktringelemente 2 und Dichtungsringe 3 wirkende, definiert vorgebbare Fügekraft einstellt. Die Relativdrehung lässt sich im einfachsten Fall mit einem mit dem Montagewerkzeug in Eingriff bringbaren Drehwerkzeug, bspw. in Form eines Schraubenschlüssels realisieren.

Die in Figur 1 illustrierte axiale Stapelanordnung 4, bestehend aus einer Anzahl von längs des stabförmigen Montagewerkzeuges 1 in abwechselnder Reihenfolge aufgefädelter elektrischer Kontaktringelemente 2 und Dichtungsringen 3, die mit Hilfe der Befestigungsmittel 5 und 6 unter Vorgabe einer axial wirkenden Fügekraft F gegenseitig kraftschlussbeaufschlagt gefügt sind, stellt ein einheitlich, manuell oder automatisch handzuhabendes Halbzeug dar, das im Weiteren mit einer erstarrungsfähigen, in fließfähiger Form vorliegenden Vergussmasse 12 in der in Figur 3 dargestellten Weise umgossen wird. Hierzu dient in vorteilhafter Weise eine Gießform 13, in die das vorstehend erläuterte Halbzeug mit den axial vorgespannten Kontaktringelementen und Dichtungsringen samt Montagewerkzeug eingelegt wird. Im Weiteren wird die Gießform 13 mit einer fließfähigen Vergussmasse 12 aufgefüllt, wobei die Vergussmasse 12 die gesamte Stapelanordnung 4, d.h. das Befestigungsmittel 6 sowie sämtliche Kontaktringelemente und Dichtungsringe umfasst, und vorzugsweise zumindest teilweise auch das Befestigungsmittel 5 umgibt.

Nach Erstarren der Vergussmasse 12 wird das Montagewerkzeug 1 entfernt, siehe Figur 4, wobei das Befestigungsmittel 6 sowie die axiale Stapelanordnung 4 in der Vergussmasse 12 verbleiben. Sollte das Befestigungsmittel 5 nicht einstückig, wie in Figur 1 dargestellt, sondern ebenfalls, wie das Befestigungsmittel 6 lösbar fest am Montagewerkzeug montiert sein, so kann in diesem Fall auch das Befestigungsmittel 5 in der ausgehärteten Vergußmasse verbleiben.

Um zu verhindern, dass das Montagewerkzeug 1 mit seinem über das Innengewinde 18 des Befestigungsmittels 6 hinausragenden Ende keine feste Stoffschlussverbindung mit der erstarrten Vergußmasse 12 eingeht, wodurch ein Lösen des Montagewerkzeuges 1 zumindest erschwert würde, wird das Ende des Montagewerkzeuges vor dem Vergiessen mit einer Schutzmasse 19, siehe Figur 2a benetzt, bspw. Silikon o.ä..

Nach Entfernen des Montagewerkzeuges 1 bleibt die zwischen den Kontaktringelementen 2 und Dichtungsringen 1 vorherrschende axiale Fügekraft F unverändert erhalten, zumal die Fügekraft F von der erstarrten Vergussmasse 12 abgefangen wird. Im Unterschied zu dem in Figur 3 illustrierten Herstellungsschritt, bei dem die Fügekraft F beidseitig von den Befestigungsmitteln 5 und 6 erzeugt und abgefangen wird, wird die Fügekraft F im Falle der Figur 4 nach Entfernen des Montagewerkzeuges 1 ausschließlich von der erstarrten Vergussmasse 12 abgestützt bzw. abgefangen und bleibt aufgrund der Formstabilität der erstarrten Vergußmasse unverändert erhalten.

Zugleich bildet sich nach Entfernen des Montagewerkzeuges 12 innerhalb des sich durch Erstarren der Vergussmasse ausbildenden Kopfteils 14 ein freier Zugang 15 innerhalb eines Hohlraumes, in den eine nicht weiter illustrierte Steckeranordnung zur elektrischen Kontaktierung der einzelnen elektrischen Kontaktringelemente 2 einführbar ist.

Von jedem elektrisch leitenden Kontaktringelemente 2 geht ferner eine elektrische Leiterstruktur 16 ab, die das Kopfteil 14 einseitig durchragt und mit elektrischen Komponenten innerhalb einer implantierbaren medizinischen Vorrichtung 17 verbunden sind. Hierzu werden die elektrischen Leiterstrukturen entweder bereits vor dem Vergießen des Halbzeuges mit den jeweiligen elektrischen Kontaktringelementen verbunden und gemeinsam mit dem beschriebenen Halbzeug vergossen. Ebenso ist es möglich die elektrischen Leiterstrukturen nachträglich durch Einbringen von Bohrlöchern in die ausgehärtete Matrix und entsprechendes Einfügen der Leiterstrukturen in die Bohrlöcher mit den Kontaktringelementen zu kontaktieren.

Die implantierbare medizinische Vorrichtung 17 ist, wie eingangs erwähnt, vorzugsweise ein eingehauster Pulsgenerator zur Erzeugung elektrischer Stimulationssignale, die über eine in Figur 4 nicht dargestellte Steckereinheit und damit verbundenen elektrischen Leitungen an bestimmte intrakorporale Bereiche applizierbar sind. Das Kopfteil 14 wird typischerweise lösbar fest an die medizinische Vorrichtung 17 gefügt, so dass ein Austausch bspw. des Pulsgenerators möglich ist ohne die intrakorporal verorteten elektrischen Leitungen zu beeinträchtigen.

### Bezugszeichenliste

- 1: stabförmig ausgebildetes Montagewerkzeug
- 2: elektrisch leitendes Kontaktringelement
- 3.: elektrisch isolierender Dichtungsring
- 4: Stapelanordnung
- 5, 6: Befestigungsmittel
- 7: Hülse
- 8: Bohrung mit Innengewinde
- 9: Madenschraube
- 10: Mutter
- 10': Plattenelement
- 11: Außengewinde
- 12: Vergussmasse
- 13: Gießform
- 14: Kopfteil
- 15: Hohlraum, freier Zugang
- 16: elektrische Leiterstruktur
- 17: implantierbare medizinische Vorrichtung
- 18: Innengewinde
- 19: Schutzmasse
- D: Drehung
- F: Fügekraft

## Patentansprüche

1. Verfahren zum Herstellen eines Kopfteils (14) einer implantierbaren medizinischen Vorrichtung (17), mit einem Kopfteilgehäuse, das eine sacklochartige Ausnehmung besitzt, längs der in koaxialer Anordnung und axial serieller Abfolge wenigstens ein elektrisch leitendes Kontaktringelement (2) sowie ein elektrisch isolierender, elastisch verformbarer Dichtungsring (3) unter axialer Fügekraft gegeneinander kraftschlüssig gefügt sind, wobei das wenigstens eine Kontaktringelement (2) sowie der wenigstens eine Dichtungsring (3) längs eines stabförmigen Montagewerkzeuges (1) angeordnet und längs des stabförmigen Montagewerkzeuges (1) durch Erzeugen einer Fügekraft (F) gegeneinander kraftschlüssig gefügt werden, und wobei zumindest das wenigstens eine längs des stabförmigen Montagewerkzeuges (1) angeordnete, kraftschlüssig gefügte Kontaktringelement (2) sowie der wenigstens eine Dichtungsring (3) mit einer erstarrungsfähigen, in fließfähiger Form vorliegenden Vergußmasse (12) ummantelt werden, wobei das Erzeugen der Fügekraft (F) zwischen dem wenigstens einen Kontaktringelement (2) und dem Dichtungsring (3) längs des Montagewerkzeuges (1) mittels beidseitig zum wenigstens einen Kontaktringelement (2) und Dichtungsring (3) längs des Montagewerkzeuges (1) angebrachter Befestigungsmittel (5, 6) durchgeführt wird,
**dadurch gekennzeichnet, dass** wenigstens ein Befestigungsmittel (6) axialbeweglich und lösbar axialfest am Montagewerkzeug (1) fixiert wird und in Art einer Mutter mit Innengewinde ausgebildet ist, das in Eingriff mit einem längs des Montagewerkzeuges (1) vorgesehenen Außengewinde (11) derart gebracht wird, so dass das in Art einer Mutter ausgebildete Befestigungsmittel (6) durch relatives Verdrehen längs des Außengewindes (11) des Montagewerkzeuges (1) einseitig kraftschlüssig unter Ausbildung der Fügekraft (F) in Eingriff mit dem wenigstens einen Kontaktringelement (2) oder Dichtungsring (3) gebracht wird,
dass das stabförmige Montagewerkzeug (1) von dem wenigstens einen Kontaktringelement (2) sowie dem wenigstens einen Dichtungsring (3) nach Erstarren der Vergußmasse (12), die in Form einer zumindest einen Teil des Kopfteilgehäuses bildenden, formstabilen Matrix die axiale Fügekraft (F) mechanisch abstützt, gelöst und entfernt wird, und dass
das wenigstens eine Befestigungsmittel (6) in der verfestigten Vergußmasse (12) verbleibt.

2. Verfahren nach Anspruch 1,
**dadurch gekennzeichnet, dass** eine Vielzahl von in seriell abwechselnder Reihenfolge längs des stabförmigen Montagewerkzeuges (1) angeordnete Kontaktringelemente (2) und Dichtungsringe (3) vorgesehen wird.

3. Verfahren nach Anspruch 1 oder 2,
**dadurch gekennzeichnet, dass** vor oder nach dem Ummanteln und Erstarren der Vergußmasse (12) das wenigstens eine Kontaktringelement (2) mit wenigstens einer elektrischen Leiterstruktur (16) verbunden wird, die durch die Vergußmasse (12) hinaus führt.

4. Verfahren nach einem der Ansprüche 1 bis 3,
**dadurch gekennzeichnet, dass** das Ummanteln zumindest des einen längs des stabförmigen Montagewerkzeuges (1) angeordneten kraftschlüssig gefügten Kontaktringelementes (2) sowie Dichtungsringes (3) unter Verwendung einer Gießform (13) erfolgt.

5. Verfahren nach einem der Ansprüche 1 bis 4,
**dadurch gekennzeichnet, dass** nach dem Lösen und Entfernen des stabförmigen Montagewerkzeuges (1) von dem wenigstens einen Kontaktringelement (2) sowie dem Dichtungsring (3) ein freier Zugang zu einem von dem wenigstens einen Kontaktringelement (2) sowie dem Dichtungsring (3) umschlossenen zylinderförmigen Volumen innerhalb des Kopfteils (14) gebildet wird.

6. Verfahren nach einem der Ansprüche 1 bis 5,
**dadurch gekennzeichnet, dass** das Kopfteil (14) ausgebildet wird zur mechanischen und elektrischen Ankoppelung an eine als Pulsgenerator ausgebildete implantierbare medizinische Vorrichtung (17).

## Claims

1. Method of producing a head part (14) of an implantable medical device (17) with a head part housing, which has a blind hole-like recess, along which in coaxially arrangement and axially serial sequence, at least one electrically conductive contact ring element (2) as well as an electrically insulating, elastically deformable sealing ring (3) are joined together in a non-positive manner through an axial joining force, wherein the at least one contact ring element (2) and the at least one sealing ring (3) are arranged along a rod-shaped assembly tool (1) and along the rod-shaped assembly tool (1) are non-positively joined together with each other through the production of a joining force (F), and wherein the at least one non-positively joined contact ring element (2) arranged along the rod-shaped assembly tool (1) as well as the at least one sealing ring (3) are surrounded by a solidifiable casting compound (12) present in flowable form, wherein the joining force (F) between the at least one contact ring element (2) and the sealing ring (3) is produced along the assembly tool (1) by way of fastening means (5, 6) applied on at least both sides of the at least one contact ring element (2) and sealing ring (3) along the assembly tool (1),
**characterised in that** at least one fastening means (6) is fixed in an axially moveable and detachable axially firm manner on the assembly tool (1) and is configured in the manner of a nut with an internal thread which is brought into contact with an outer thread (11) provided along the assembly tool (1) in such a way that the fastening means (6) designed in the manner of a nut, through relative turning along the outer thread (11) of the assembly tool (1), is on one side non-positively brought into engagement with the at least one contact ring element (2) or sealing ring (3) while producing the joining force (F),
**in that** after solidification of the casting compound (12), which in the form of a dimensionally stable matrix forming part of the head part housing mechanically supports the axial joining force (F), the rod shaped assembly tool (1) is detached and removed from the at least one contact ring element (2) and the at least one sealing ring (3), and **in that**
the at least one fastening means (6) remains in the solidified casting compound (12).

2. Method according to claim 1,
**characterised in that** a plurality of contact ring elements (2) and sealing rings (3) is provided in serially alternating sequence along the rod-shaped assembly tool (1).

3. Method according to claim 1 or 2
**characterised in that** before and after being surrounded with and solidification of the casting compound (12), the at least one contact ring element (2) is combined with at least one electrical conductor structure (16) which lead outs through the casting compound (12).

4. Method according to any one of claims 1 to 3
**characterised in that** surrounding of the at least one contact ring element (2) as well as sealing ring (3) arranged non-positively joined along the rod-shaped assembly tool (1), takes place using a casting mould (13) .

5. Method according to any one of claims 1 to 4,
**characterised in that** after detaching and removing the rod-shaped assembly tool (1) from the at least one contact ring element (2) as well as the sealing ring (3), a free access is created to a cylindrical volume within the head part (14) surrounded by the at least one contact ring element (2) as well as the sealing ring (3).

6. Method according to any one of claims 1 to 5,
**characterised in that** the head part (14) is designed for mechanical and electrical connection to an implantable medical device (17) configured as a pulse generator.

## Revendications

1. Procédé de fabrication d'une partie tête (14) d'un dispositif médical implantable (17), avec un boîtier de partie tête, qui possède un évidement en forme de trou borgne, le long duquel en disposition coaxiale et en succession axialement en série au moins un élément annulaire de contact électroconducteur (2) ainsi qu'une bague d'étanchéité d'isolation électrique, élastique, déformable (3), sont posés l'un contre l'autre par conformité de force en exerçant une force d'assemblage, sachant qu'au moins un élément annulaire de contact (2) ainsi qu'au moins une bague d'étanchéité (3) sont disposés le long d'un outil de montage en forme de tige (1) et sont assemblés l'un contre l'autre par conformité de force le long d'un outil de montage en forme de tige (1) en exerçant une force d'assemblage (F) et sachant qu'au moins un élément annulaire de contact (2) assemblé par conformité de force, disposé le long de l'outil de montage en forme de tige (1) ainsi qu'au moins une bague d'étanchéité (3) sont enrobés avec une masse de remplissage (12) solidifiable, présente sous forme fluide, sachant que la production de la force d'assemblage (F) entre au moins l'élément annulaire de contact (2) et la bague d'étanchéité (3) le long de l'outil de montage (1) est exercée au moyen de moyens de fixation (5, 6) placés des deux côtés d'au moins un élément annulaire de contact (2) et d'une bague d'étanchéité (3) le long de l'outil de montage (1),
**caractérisé en ce qu'**au moins un moyen de fixation (6) est fixé axialement mobile et de façon amovible en position axiale fixe à 'outil de montage (1) et est constitué sous la forme d'un écrou avec un filetage intérieur, qui est mis en prise avec un filetage extérieur (11) prévu le long de l'outil de montage (1) de telle sorte que le moyen de fixation (6) constitué sous la forme d'un écrou est mis en prise unilatéralement par conformité de force en constituant la force d'assemblage (F) avec au moins un élément annulaire de contact (2) ou une bague d'étanchéité (3) par rotation relative le long du filetage extérieur (11) de l'outil de montage (1),
**en ce que** l' outil de montage en forme de tige (1) est défait et enlevé d'au moins un élément annulaire de contact (2) ou d'au moins une bague d'étanchéité (3) après solidification de la masse de remplissage (12), qui soutient mécaniquement la force d'assemblage axiale (F) sous la forme d'une matrice de forme stable, formant une partie du boîtier de la partie tête et
**en ce qu'**au moins un moyen de fixation (6) reste dans la masse de remplissage (12) solidifiée.

2. Procédé selon la revendication 1,
**caractérisé en ce qu'**il est prévu une pluralité d'éléments annulaires de contact (2) et de bagues d'étanchéité (3) disposés dans en ordre alternatif en série le long de l'outil de montage (1).

3. Procédé selon la revendication 1 ou 2,
**caractérisé en ce qu'** avant ou après l'enrobage et la solidification de la masse de remplissage (12), au moins un élément annulaire de contact (2) est relié à au moins une structure conductrice électrique (16), qui passe au-delà à travers la masse de remplissage (12).

4. Procédé selon l'une quelconque des revendications 1 à 3,
**caractérisé en ce que** l'enrobage d'au moins un élément annulaire de contact (2) ainsi que d'une bague d'étanchéité (3) assemblés par conformité de force disposé le long de l'outil de montage en forme de tige (1) a lieu en utilisant un moule (13).

5. Procédé selon l'une quelconque des revendications 1 à 4,
**caractérisé en ce qu'**après le démontage et l'enlèvement de l'outil de montage en forme de tige (1) d'au moins un élément annulaire de contact (2) et de la bague d'étanchéité (3), un libre accès est formé à un volume cylindrique à l'intérieur de la partie tête (14), entouré par au moins un élément annulaire de contact (2) ainsi que par la bague d'étanchéité (3).

6. Procédé selon l'une quelconque des revendications 1 à 5,
**caractérisé en ce que** la partie tête (14) est constituée pour le couplage mécanique et électrique à un dispositif médical implantable (17) constitué sous la forme d'un générateur d'impulsions.
